# EUROPEAN PATENT SPECIFICATION

(11) **EP 0 984 808 B1**
(45) Date of publication and mention of the grant of the patent: **30.06.2004**
(21) Application number: 98919479.0
(22) Date of filing: 30.04.1998
(51) Int. Cl.: A61M 25/06

(54) **INTRAVENOUS INFUSION DEVICE**
INTRAVENÖSE INFUSIONSVORRICHTUNG
DISPOSITIF DE PERFUSION INTRAVEINEUSE

(30) Priority: 30.04.1997 IT BO970261; 30.01.1998 IT BO980038
(43) Date of publication of application: 15.03.2000
(73) Proprietor: Bragaglia, Michela, 40036 Monzuno (IT)
(72) Inventor: BRAGAGLIA, Michela, I-40141 Bologna (IT); BRAGAGLIA, Giorgio, DECEASED (IT)
(74) Representative: Lanzoni, Luciano
(86) International application number: PCT/IT1998/000111
(87) International publication number: WO 1998/048883

(56) References cited:
- WO-A-81/01518
- WO-A-88/07388
- US-A- 4 923 446
- US-A- 5 498 241

## Description

### TECHNICAL FIELD

The present invention relates to an intravenous infusion device.

The present invention is designed to provide a simple effective solution to the problem, frequently and long encountered by medical workers, of accidental pricking by intravenous infusion needles.

### BACKGROUND ART

At present, intravenous infusion devices substantially comprise a needle fitted simply with a cap which is removed and disposed of prior to use, and no valid protection has yet been devised to protect the needle after use.

As is known, the needle may become infected during use, and is therefore a particularly dangerous vehicle for the direct transmission of infectious diseases.

Nor do the precautions recommended when removing the needle from the patient's body offer any guarantee of safety; which precautions normally consist in disposing of the entire set or inserting the needle in the drip bottle seal, etc. Either case, however, involves disposing of an unprotected needle, which must be gripped firmly by the two wings between the thumb and fingers of one hand, while, with the other hand, the user replaces the needle very carefully inside the cap.

As is known, for example from US-A-5 498 241, during infusion, the two wings, which form what is commonly referred to as a "butterfly", are attached by a plaster to the patient's skin; and, to withdraw the needle from the vein after infusion, the two wings are folded one against the other, which is a fairly traumatic operation on account of the needle rotating back and forth inside the vein as the wings are folded.

The intravenous infusion device according to the present invention operates in substantially the same way as known devices, but, in addition to solving the aforementioned safety problems, also provides for easier insertion of the needle into the vein to minimize discomfort to the patient.

As is known, if inserted incorrectly, for example with the cutting edges facing downwards as opposed to upwards, the needle is extremely difficult to insert inside the vein. Using conventional infusion devices, correct orientation of the cutting edges at the tip of the needle must therefore be determined each time, which is not only time-consuming but also fairly unreliable.

This drawback is eliminated by the device according to the invention, in which, when the single wing is gripped, the cutting edges at the tip of the needle are so oriented as to ensure correct, smooth, painless insertion into the vein. That is, the inclined surface defining the tip of the needle is located on the same side of the needle as the single wing, so that correct orientation of the tip of the needle need no longer be determined by eye.

### DISCLOSURE OF INVENTION

According to the present invention, there is provided an intravenous infusion device as defined in claim 1.

### BRIEF DESCRIPTION OF DRAWINGS

A non-limiting embodiment of the intravenous infusion device according to the present invention will be described purely by way of example with reference to the accompanying drawings, in which:
Figure 1 shows a partially sectioned side view of the device before and after use;
Figure 2 shows a front view of the device;
Figure 3 shows a partially sectioned side view of the device showing insertion of the needle inside the cap at the assembly stage;
Figure 4 shows a partially sectioned side view of the device as used;
Figures 5, 5a, 5b respectively show a plan view, a front view and a longitudinal section of the slot in the cap.

### BEST MODE FOR CARRYING OUT THE INVENTION

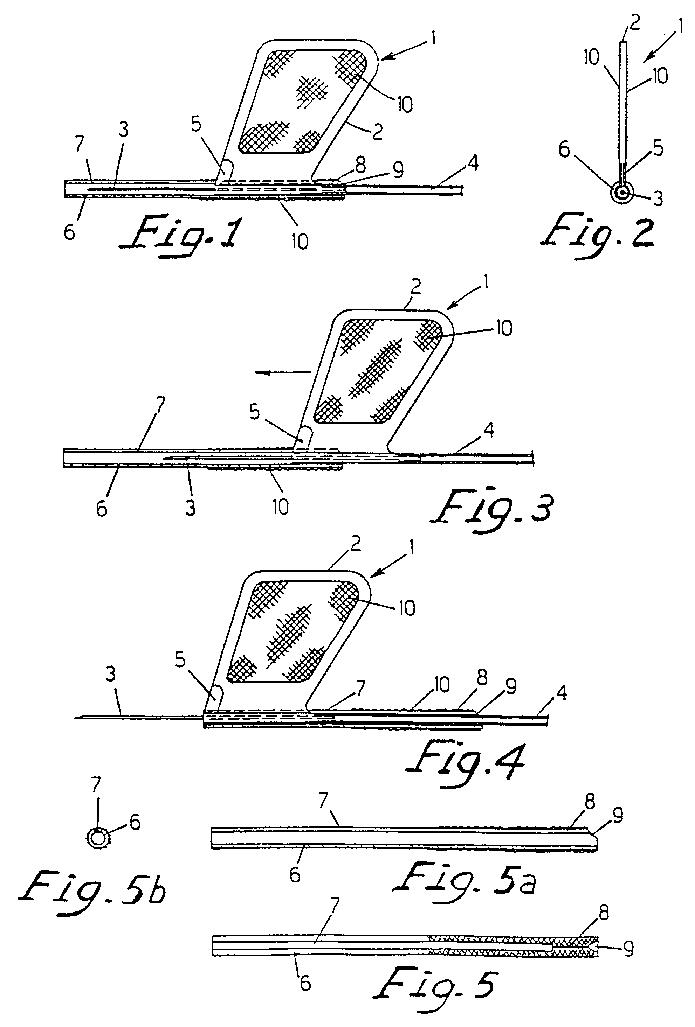

Figure 1 shows an intravenous infusion device 1 as removed from a sterile set, and which substantially comprises a single wing 2 (Figure 2) integral with a base by which to secure a needle 3 to a feed or withdrawal tube 4. The front edge of wing 2, at needle 3, has a bevel 5 similar to the bow of a ship.

The above are fitted externally in sliding manner with a cap 6 defined by a tube of plastic material, appropriately longer than needle 3, and having a longitudinal slot 7 (Figures 5, 5a, 5b) in which wing 2 slides, and which opens out at the rear end with a clean cut 8 and a lead-in bevel 9 diverging outwards. Slot 7 may be so sized that wing 2 slides inside it with a given amount of friction; may be narrower at the ends to better secure cap 6 to the structure of needle 3 in the fully withdrawn or fully inserted position of the needle; and may be either straight, as in the drawings, or substantially helical (not shown).

Needle 3 together with wing 2 may be inserted inside cap 6 through the open front end of slot 7, or inside the rear end of cap 6 (Figure 3) through bevel 9 and clean cut 8, using bevel 5 of wing 2. If the latter method is used, the front end of slot 7 may be closed (not shown).

For better grip by the operator, both cap 6 and wing 2 have knurled portions 10, which may be formed on both sides of wing 2.

Figure 4 shows device 1 ready for use, with needle 3 projecting from cap 6. The cutting edges at the tip of needle 3 are so oriented with respect to wing 2 that, when the wing is gripped by the operator, the cutting edges provide for correct, painless insertion into the vein. That is, the cutting edges of needle 3 are located on the same side of needle 3 as wing 2.

Wing 2 is obviously secured in place with a plaster in the usual way, and may be laid on the right or left arm in exactly the same way as for currently marketed devices.

After infusion, needle 3 is withdrawn more easily than at present : as opposed to folding two wings one against the other, the single wing 2 is rotated roughly 90° with respect to the body surface, and needle 3 is immediately covered and protected by cap 6, which is fitted on to needle 3 as shown in Figure 1.

The above operation is made easier and safer by the knurled portions 10 on both sides of wing 2 and on cap 6.

## Claims

1. An intravenous infusion device (1) comprising:
- a needle (3) connectable to a flexible infusion tube (4);
- fastening means (2) for fastening said needle (3) to the patient's body; and
- means (6) for protecting and covering said needle (3);
wherein said means (6) for protecting and covering said needle (3) comprise a substantially cylindrical element (6) having a longitudinal slot (7) in which said fastening means (2) slide; and wherein said needle (3) being movable by the operator from a withdrawn position fully covering and protecting the needle (3), to a forward position exposing the needle (3) for use, so as to provide, by means of the same element (6), for protection of said needle (3) prior to use, and for protection of said needle (3) after the use;
the device (1) being **characterized in that** said fastening means (2) for fastening said needle (3) to the patient's body comprise a single wing (2);
and **in that** the inclined surface defining the tip of said needle (3) is located on the same side of the needle as the single wing (2), so that correct orientation of the tip a said needle (3) need no longer be determined by eye.

2. . A device (1) as claimed in Claim 1, wherein said slot (7) and said single wing (2) are formed as to slide frictionally with respect to each other.

3. . A device (1) as claimed in one of the foregoing Claims, wherein said slot (7) of said element (6) is substantially straight.

4. A device (1) as claimed in Claim 1 or 2, wherein said slot (7) of said element (6) is substantially helical.

5. . A device (1) as claimed in one of the foregoing Claims, wherein said single wing (2) and said element (6) have, on at least one side of the gripping surfaces, knurled portions (10) for ensuring better grip.

6. A device (1) as claimed in one of the foregoing Claims, wherein said needle (3) is inserted inside said slot (7) through an open end of said slot (7).

7. . A device (1) as claimed in Claim 6, wherein said single wing (2) has a bottom bevel (5) for simplifying insertion of the single wing (2) inside said slot (7) through the open end.

8. . A device (1) as claimed in Claim 6 or 7, wherein the open end is formed by a clean cut (8).

## Patentansprüche

1. Intravenöse Infusionsvorrichtung (1), enthaltend:
- eine Nadel (3), die an einen flexiblen Infusionsschlauch (4) anschliessbar ist;
- Befestigungsmittel (2) zum Befestigen der genannten Nadel (3) an dem Körper des Patienten; und
- Mittel (6) zum Schützen und Abdecken der genannten' Nadel (3);
wobei die genannten Mittel (6) zum Schützen und Abdecken der genannten Nadel (3) ein im wesentlichen zylindrisches Element (6) einen im wesentlichen länglichen Schlitz (7) enthalten, in welchem die genannten Befestigungsmittel (2) gleiten; und wobei die genannte Nadel (3) durch den Benutzer aus einer zurückgezogenen Position, in welcher die Nadel (3) vollkommen verdeckt und geschützt ist, in eine vorgeschobene Position zum Benutzen der Nadel (3) beweglich ist, so dass mit Hilfe desselben Elementes (6) ein Schutz für die genannte Nadel (3) vor der Benutzung und ein Schutz der genannten Nadel (3) nach der Benutzung vorgesehen wird;
und wobei die Vorrichtung (1) **dadurch gekennzeichnet ist, dass** die genannten Befestigungsmittel (2) zum Befestigen der genannten Nadel (3) am Körper des Patienten einen einzigen Flügel (2) enthalten;
und dass die schräge, die Spitze der genannten Nadel (3) bildende Oberfläche auf derselben Seite der Nadel wie der einzige Flügel (2) angeordnet ist, so dass die korrekte Ausrichtung der Spitze einer genannten Nadel (3) nicht mehr länger durch das Auge bestimmt wird.

2. Vorrichtung (1) nach Patentanspruch 1, bei welcher der genannte Schlitz (7) und der genannte einzige Flügel (2) so ausgebildet sind, dass sie reibschlüssig im Verhältnis zueinander gleiten.

3. Vorrichtung (1) nach einem der vorstehenden Patentansprüche, bei welcher der genannte Schlitz (7) des genannten Elementes (6) im wesentlichen geradlinig ist.

4. Vorrichtung (1) nach Patentanspruch 1 oder 2, bei welcher der genannte Schlitz (7) des genannten Elementes (6) im wesentlichen schneckenförmig ist.

5. Vorrichtung (1) nach einem der vorstehenden Patentansprüche, bei welcher der genannte einzige Flügel (2) und das genannte Element (6) wenigstens auf einer Seite der Greifflächen gerändelte Abschnitte (10) zum Sichern eines besseren Griffes haben.

6. Vorrichtung (1) nach einem der vorstehenden Patentansprüche, bei welcher die genannte Nadel (3) in den genannten Schlitz (7) durch ein offenes Ende des genannten Schlitze (7) eingesetzt wird.

7. Vorrichtung (1) nach Patentanspruch 6, bei welcher der genannte einzige Flügel (2) eine untere Abschrägung (5) zum Vereinfachen des Einsetzens des einzigen Flügels (2) in den genannten Schlitz (7) durch das offene Ende aufweist.

8. Vorrichtung (1) nach Patentanspruch 6 oder 7, bei welcher das offene Ende durch einen glatten Schnitt (8) gebildet ist.

## Revendications

1. Un dispositif de perfusion intraveineuse (1) comprenant :
- une aiguille (3) pouvant être raccordée à un tuyau flexible (4) de perfusion ;
- des moyens de fixation (2) pour fixer ladite aiguille (3) au corps du patient ; et
- des moyens (6) pour protéger et couvrir ladite aiguille (3) ;
où lesdits moyens (6) de protection et de couverture de ladite aiguille (3) comprennent un élément (6), essentiellement cylindrique, comportant une fente longitudinale (7) dans laquelle coulissent lesdits moyens de fixation (2) ; et où ladite aiguille (3) peut être déplacée par l'opérateur d'une position rentrée, dans laquelle l'aiguille (3) est entièrement recouverte et protégée, à une position sortie, dans laquelle l'aiguille (3) est exposée pour être utilisée, de manière à pouvoir, par l'intermédiaire de ce même élément (6), protéger ladite aiguille (3) avant utilisation et après utilisation ; ledit dispositif (1) étant **caractérisé en ce que** lesdits moyens de fixation (2) destinés à fixer ladite aiguille (3) au corps du patient sont constitués par une seule ailette (2) ; et **en ce que** la surface inclinée définissant le bout de ladite aiguille (3) est située du même côté de l'aiguille que l'ailette (2), de sorte que l'orientation appropriée du bout de ladite aiguille (3) n'a plus besoin d'être déterminée visuellement.

2. Le dispositif (1) selon la revendication 1, **caractérisé en ce que** ladite fente (7) et ladite ailette (2) sont conçues de manière à coulisser avec frottement l'une par rapport à l'autre.

3. Le dispositif (1) selon l'une des revendications précédentes, **caractérisé en ce que** ladite fente (7) de l'élément (6) susmentionné est essentiellement rectiligne.

4. Le dispositif (1) selon la revendication 1 ou 2, **caractérisé en ce que** ladite fente (7) de l'élément (6) susmentionné est essentiellement hélicoïdale.

5. Le dispositif (1) selon l'une des revendications précédentes, **caractérisé en ce que** ladite ailette (2) et ledit élément (6) ont, sur au moins un côté des surfaces de prise, des portions moletées (10) destinées à assurer une meilleure prise.

6. Le dispositif (1) selon l'une des revendications précédentes, **caractérisé en ce que** ladite aiguille (3) est introduite à l'intérieur de ladite fente (7) à travers une extrémité ouverte de cette même fente (7).

7. Le dispositif (1) selon la revendication 6, **caractérisé en ce que** ladite ailette (2) présente un biseau inférieur (5) destiné à faciliter l'introduction de cette même ailette (2) à l'intérieur de ladite fente (7) à travers l'extrémité ouverte.

8. Le dispositif (1) selon la revendication 6 ou 7, **caractérisé en ce que** l'extrémité ouverte est définie par une coupe nette (8).
